# EUROPÄISCHE PATENTANMELDUNG

(11) **EP 0 904 838 A2**
(43) Veröffentlichungstag der Anmeldung: **31.03.1999**
(21) Anmeldenummer: 98115040.2
(22) Anmeldetag: 11.08.1998
(51) Int. Cl.: B01J 37/02, B01J 23/38

(54) **Verfahren zur Herstellung eines Schalenkatalysators**

(30) Priorität: 30.09.1997 DE 19743100
(71) Anmelder: DEGUSSA AG, 60311 Frankfurt (DE)
(72) Erfinder: Müller, Herbert, Dr., 63755 Alzenau (DE); Bösing, Stefan, 55246 Mainz-Kostheim (DE); Behl, Walter, 63776 Mömbris (DE)

(57) **Zusammenfassung**

Verfahren zur Herstellung eines Schalenkatalysators, bei dem ein nichtporöses anorganisches Trägermaterial, welches eine BET-Oberfläche von weniger als 80 m²/g aufweist, mit einer katalytisch wirksamen Schale versehen wird, dadurch gekennzeichnet, daß zunächst in einem Schritt eine Suspension aus wenigstens einer in Wasser löslichen Edelmetallverbindung und einer im wesentlichen nicht wasserlöslichen Coatingverbindung auf das Trägermaterial aufgetrocknet wird und anschließend das resultierende schalenförmig beschichtete Trägermaterial in einem reduktiv wirksamen Gasstrom aktiviert wird.

Mit dem Verfahren der Erfindung gelingt es, ein nicht poröses Trägermaterial in einem einzigen Arbeitsschritt mit Aktiv- und Coatingmaterial gleichzeitig zu beschichten sowie mit dem resultierenden Schalenkatalysator höhere Raumgeschwindigkeiten ohne Umsatzeinbußen bei der selektiven Hydrierung von Acetylen in Gasströmen zu erzielen.

## Beschreibung

Die Erfindung betrifft ein Verfahren zur Herstellung eines Schalenkatalysators, bei welchem ein nicht poröses anorganisches Trägermaterial mit niedriger Oberfläche von weniger als 80 m² pro g gemessen nach BET mit einer katalytisch wirksamen Schale versehen wird.

Metalle, vorzugsweise Edelmetalle, werden trägergebunden in elementarer Form oder als Oxide in vielen technischen Verfahren zur Katalyse eingesetzt. Als Katalysatorträger kann man poröse oder nicht poröse Materialien einsetzen.

Bei der Imprägnierung von Trägermaterialien mit wäßrigen Metallsalzlösungen und anschließender Reduktion des Salzes kann das Metall auf den Träger in Form einer Schale, welche den Träger vollkommen einhüllt, abgeschieden werden. Von diesen Schalenkatalysatoren ist bekannt, daß sie u. a. für Selektivhydrierungen und Oxidationsreaktionen mit Sauerstoff in der Gasphase besonders geeignet sind.

Ein Anwendungsbereich von besonderem Interesse umfaßt dabei die selektive Hydrierung von Acetylen, insbesondere die Reinigung eines Chlorwasserstoffgasstroms innerhalb des Oxychlorierungsprozesskreislaufes zur Herstellung von Vinylchlorid.

Vinylchlorid wird entweder rein thermisch oder in Gegenwart von Katalysatoren bei höherer Temperatur aus 1,2-Dichlorethan unter Abspaltung von Chlorwasserstoff hergestellt. Nach Abtrennung der Hauptmenge des Vinylchlorids fällt ein Chlorwasserstoffgas an, das im Regelfall bis zu 3000 Vppm Acetylen enthält. Vor der Rückführung dieses Chlorwasserstoffs in die Oxychlorierung ist es erforderlich, in einer Zwischenstufe, den störenden Acetylengehalt des Gasgemisches möglichst weitgehend zu entfernen. Dies geschieht durch katalytische Hydrierung, wobei die wesentliche Anforderung die möglichst weitgehende Entfernung des Acetylens (Restacetylengehalt < 30 Vppm) durch selektive Hydrierung desselben zu im Oxychlorierungsprozess verwertbaren Ethylen ist.

Die Verfahrensbedingungen für die Reinigung des beschriebenen Chlorwasserstoffgasstroms sind in EP 0052271 B1 und US 4388278 ausführlich beschrieben. Üblicherweise wird mit einem deutlichen Wasserstoffüberschuß im einem Temperaturbereich von 120-180°C in Druckbereichen von 6 bis 20 bar abs. hydriert.

Für die Reinigung des Chlorwassergasstroms durch selektive Hydrierung des in ihm enthaltenen Acetylens kommen Festbettkatalysatoren zum Einsatz. Es handelt sich dabei um vorzugsweise Palladium-haltige Katalysatoren. Katalysatorformulierungen auf Aluminiumoxid sind für diesen Einsatzbereich bekannt aber in ihrem Leistungspotential / Selektivität und Beständigkeit eingeschränkt.

Im Patent DE 30 37 047 C2 ist ein Festbettkatalysator beschrieben, der dadurch gekennzeichnet ist, daß der Katalysator durch Imprägnieren von Siliciumcarbid als Trägermaterial mit einer Lösung eines Palladiumsalzes, sowie Trocknen und Reduzieren des Palladiumsalzes mit Wasserstoff hergestellt wird. Dieser Katalysator ist auf einem vergleichsweise teuren Trägermaterial präpariert.

Die EP-O 576 944 A1 offenbart durch Abscheidung einer Legierung durch PVD (physical vapor deposition) und/oder chemical vapor deposition (CVD) auf einem Formkörper hergestellte Schalenkatalysatoren.

Als Träger kommen nicht poröse Formkörper aus Glas, Quarzglas, Keramik, Titandioxid, Zirkondioxid, Aluminiumoxid, Aluminiumsilikate, Borate, Stealit, Magnesiumsilikat, Siliciumdioxid, Silicaten, Metall, Kohlenstoff, z. B. Graphit oder Mischungen dieser Materialien in Frage. Die auf den Formkörper aufgebrachte Legierungsschicht enthält mindestens ein Metall, das bevorzugt sehr leicht oxidierbar ist, wie Silicium, Aluminium, Zirkon oder Titan. Die Schichtdicke liegt im Bereich von 100 nm bis 50 µm.

Schließlich beschreibt die EP 0 755 718 A1 ein Verfahren zur Herstellung beladener nicht poröser Trägermaterialien, bei dem sich nicht poröse anorganische Trägermaterialien abriebsfest mit Metall und/oder Metalloxyd beschichten lassen, indem man hochverdünnte wäßrige Lösungen mindestens einer Metallverbindung auf die erhitzten Formkörper aufbringt, so daß das Wasser sofort verdampfen kann, und anschließend bei erhöhter Temperatur ggf. kalziniert.

Aus der DE 32 00 483 A1 ist ein Verfahren zur Herstellung kieselsäurehaltiger Formkörper bekannt, bei dem man auf eine anorganische Grundmasse eine Mischung aus löslicher und nicht löslicher Kieselsäure aufbringt. Die resultierenden Formkörper können als Träger für verschiedene Katalysatormaterialien Anwendung finden.

Es wird für den beschriebenen Anwendungsfall der Chlorwasserstoffgasreinigung durch selektive Acetylenhydrierung auch ein Katalysator beschrieben, der als Trägermaterial Siliciumdioxid verwendet (Chem.-Ing.-Tech. 59 (1987) Nr.8 ,S. 645-647). Dieser Katalysator ist, wie die anderen Formulierungen, bezüglich seines Leistungsvermögens (Raumgeschwindigkeitsbelastung) limitiert.

Ausgehend von diesem Stand der Technik war es eine Aufgabe der Erfindung ein Verfahren zur Herstellung eines Schalenkatalysatos der eingangs genannten Art zur Verfügung zu stellen, welches auf einfache Weise die Schaffung eines Katalysators ermöglicht, der für die genannte beispielhafte Anwendung auch bei erhöhten Raumgeschwindigkeiten (GHSV > 3000/h ) eingesetzt werden kann.

Gelöst wird diese Aufgabe durch ein gattungsgemäßes Verfahren zur Herstellung eines Schalenkatalysators, welches die Merkmale des kennzeichnenden Teils des Anspruches 1 aufweist. Zweckmäßige Ausgestaltungen des erfindungsgemäßen Verfahrens sind in den auf den Hauptanspruch rückbezogenen Ansprüchen unter Schutz gestellt.

Dadurch, daß bei der Herstellung eines Schalenkatalysators, bei welcher ein nicht poröses anorganisches Trägermaterial, welches eine BET-Oberfläche von weniger als 80 m² pro g aufweist, mit einer katalytisch wirksamen Schale versehen wird, wobei zunächst beim ersten Schritt eine Suspension aus wenigstens einer in Wasser löslichen Edelmetallverbindung und einer im wesentlichen nicht wasserlöslichen Coatingverbindung auf das Trägermaterial aufgetrocknet wird und anschließend beim zweiten Schritt das resultierende schalenförmig beschichtete Trägermaterial in einem reduktiv wirksamen Gasstrom aktiviert wird, gelingt es auf einfache und nicht ohne weiteres vorhersehbare Weise ein Produkt zu schaffen, welches den vorerwähnten Zielvorstellungen entspricht.

Insbesondere kann es dabei als überraschend angesehen werden, daß es durch das Verfahren der Erfindung möglich wird, ein nicht poröses Trägermaterial in einem einzigen Arbeitsschritt mit Aktiv- und Coatingmaterial gleichzeitig zu beschichten. Diese vorteilhaft oberflächenwirksame Struktur ist gemäß der Erfindung auf überraschend einfache Weise erhältlich.

Dabei unterscheiden sich die erfindungsgemäßen Herstellungsverfahren und die im bisher bekannten Stand der Technik offenbarten Herstellungsverfahren für Schalenkatalysatoren erheblich. So wird bei der EP 0 576 944 die Abscheidung einer Edelmetallegierung durch PVD oder CVD im Hochvakuum erreicht. Hierfür sind relativ aufwendige Technologien und Apparaturen von Nöten.

Die EP 0 755 718 A1 scheidet wasserlösliche Metallverbindungen bzw. Metallsalze, bei denen der Metallgehalt bis auf 2 Gew.-% begrenzt ist, in bewegter Schüttung auf Trägermaterial ab, wobei die Randbedingung existiert, daß das sofortige Verdampfen des Lösungswasser gewährleistet sein muß. Im Gegensatz zu dem aus der EP 0 755 718 bekannten Verfahren werden gemäß der vorliegenden Erfindung Suspensionen aus einem wasserlöslichen Material und einem im wesentlichen nicht wasserlöslichen Material auf einem nicht porösen Träger aufgetrocknet. Hierdurch wird eine andere Struktur des somit erhältlichen Schalenkatalysators wahrscheinlich, in jedem Fall jedoch ist der gecoatete Schalenkatalysator besonders einfach erhältlich.

Zu den im Rahmen des erfindungsgemäßen Verfahrens zu beschichtenden nicht porösen anorganischen Trägermaterialien gehören besonders zweckmäßig Granulate, Formkörper oder keramische Träger. Gute Ergebnisse lassen sich mit keramischen Trägern, insbesondere solchen auf Aluminiumoxid und/oder Silikatbasis, erzielen. Weiterhin günstig sind die verschiedensten Aluminiumsilikate, Aluminiumoxid selbst, Siliciumcarbid mit Gehalten an Aluminiumoxid und Siliciumdioxid, Zirkondioxid mit und ohne Gehalten an Aluminiumoxid und Siliciumdioxid, Titandioxid mit und ohne Aluminiumoxid und Siliciumdioxid, beispielsweise Korund, Feldspat, Glimmer, Steatite, keramische Steinzeuge, Glas, Quarz, usw.

In einer besonderen Ausführungsform ist das Verfahren der Erfindung dadurch gekennzeichnet, daß als Trägermaterialien Formkörper aus Glas, Quarz, Keramik, Siliciumdioxid, Aluminiumoxid, Graphit, Formkohlen, Metall oder Steatit eingesetzt werden. Hiervon wiederum sind von gesteigertem Interesse Formkörper auf Basis von SiO₂ und/oder Al₂O₃.

Von hohem Interesse als Trägermaterialien im Verfahren gemäß der Erfindung sind auch SiO₂-Granulate verschiedener Körnungen zu nennen.

Die äußere Form der als Träger des erfindungsgemäß herstellbaren Schalenkatalysators einsetzbaren Materialien kann vielfältiger Natur sein. So kann es sich um Granalien, Formkörper in Form von Kugeln, Tabletten und/oder Strängen handeln. In einer zweckmäßigen Variante des erfindungsgemäßen Verfahrens werden Trägermaterialien in Form von Hohlextrudaten, Vollextrudaten, Kugeln, Granalien, Tabletten und/oder Strängen eingesetzt. Die Trägermaterialien können auch als Extrudat, als Hohlextrudat, als Röhrenabschnitt, als Ringe oder in Linsenform eingesetzt werden.

In einer besonderen Variante werden granalienförmige Materialien im Verfahren der Erfindung gecoatet. Gute bis sehr gute Schalenpräparationen wurden insbesondere auch auf keramischen Materialien (Alumosilikate) im Rahmen der Erfindung erhalten.

Je nach angestrebtem Einsatzzweck der Schalenkatalysatoren können rollfähige Trägermaterialien von Vorteil sein. In diesem Fall sind Kugeln besonders bevorzugt.

Der Teilchendurchmesser der zu beschichtenden Trägermaterialien ist an sich nicht weiter kritisch und kann über einen größeren Bereich variieren. Die Träger haben dabei vorzugsweise Teilchendurchmesser im Bereich von 0,5 bis 50 mm. Besonders bevorzugt ist der Bereich von 1 bis 20 mm.

In einer Variante ist auch der Bereich von > 1 mm ganz besonders bevorzugt. Auch Teilchendurchmesser von kleiner 0,5 mm sind mitunter in Abhängigkeit vom angesteuerten Einsatzzweck sinnvoll.

Die BET-Oberfläche der erfindungsgemäß mit einer Schale zu versehenden anorganischen Trägermaterialien ist geringer als 80 m²/g, was auf einen nicht porösen Träger schließen läßt. Ganz besonders zweckmäßig ist es, wenn Trägermaterialien mit einer BET-Oberfläche von < 30 m²/g, vorzugsweise < 10 m²/g, eingesetzt werden.

Neben der BET-Oberfläche des Trägermaterials ist auch das Porenvolumen in einer besonders zweckmäßigen Abwandlung des erfindungsgemäßen Verfahrens von einiger Bedeutung. So kennzeichnet sich eine vorteilhafte Variante des Verfahrens der Erfindung dadurch, daß Trägermaterialien mit einem Porenvolumen < 0,5 ml/g eingesetzt werden. Besonders zweckmäßig ist es, Tragermaterialien mit einem Porenvolumen < 0,1 ml/g einzusetzen.

Die nicht porösen anorganischen Trägermaterialien des erfindungsgemäß herzustellenden Schalenkatalysators verfügen in zweckmäßiger Ausgestaltung über einen sehr geringen Fe₂O₃-Gehalt. Bevorzugt sind Fe₂O₃-Gehalte von < 0,5 Gew.-% bezogen auf das Gewicht des Trägermaterials.

Bei der erfindungsgemäßen Herstellung eines Schalenkatalysators wird in einem ersten Schritt zusammen mit dem Coatingmaterial eine in Wasser lösliche Edelmetallverbindung auf das Trägermaterial aufgetrocknet. "Wasserlöslich" im Sinne der Erfindung sind dabei Verbindungen, wenn sie in einer Konzentration, berechnet als Metall, von 0,01, vorzugsweise 0,05 Gew.-% in Wasser von 30°C gelöst werden können.

In Wasser lösliche Edelmetallverbindungen umfassen dabei bevorzugt wasserlösliche Verbindungen von Ru, Rh, Pd, Ag, Os, Ir, Pt und/oder Au.

Vorzugsweise werden dabei Oxide, Hydroxide, Carbonate, Halogenide, Nitrate, Salze organischer Säuren und/oder sonstige gängige Komplexverbindungen eingesetzt, welche die Edelmetallionen enthalten. Bei den löslichen Edelmetallverbindungen kann es sich u. U. auch um Säuren, wie beispielsweise Hexachloro-palladium-Lösungen handeln. Bevorzugt verwendet werden gemäß der Erfindung Edelmetallsalzlösungen, die Palladium enthalten. Besonders bevorzugt sind Palladiumsalzlösungen, wie Palladiumchlorid und Palladiumnitratlösungen.

Grundsätzlich ist der Edelmetallgehalt der im Verfahren der Erfindung einzusetzenden löslichen Edelmetallverbindungen zwar beliebig und richtet sich zum einen beispielsweise nach der Verfügbarkeit, es ist jedoch ganz besonders hervorzuheben, daß sich das Verfahren der Erfindung in einer besonders zweckmäßigen Variante dadurch auszeichnet, daß die wasserlösliche Edelmetallverbindung als wäßrige Lösung enthaltend die Edelmetallverbindung, berechnet als Metall, in einer Konzentration > 1 Gew.-% eingesetzt wird. Noch mehr bevorzugt ist es, wenn Edelmetallösungen mit einem Gehalt von > 5 % Edelmetall, berechnet als Metall, verwendet werden. Lösungen, die einen Gehalt von < 1 % Edelmetall aufweisen, müßten in unüblich großen Mengen angewandt werden. Hieraus würde eine verlängerte Auftrocknungszeit resultieren, um einen entsprechenden Edelmetallgehalt in der Katalysatorschale zu erreichen.

Wie bereits ausgeführt, handelt es sich bei den löslichen Edelmetallverbindungen, die gemäß der Erfindung eingesetzt werden, bevorzugt um solche, die in einer Konzentration, bezogen auf Metall, von wenigstens 0,01 Gew.-% in Wasser von 30°C gelöst werden können.

Im Gegensatz dazu handelt es sich bei den im wesentlichen nicht in Wasser löslichen Coatingverbindungen, die gleichzeitig mit der wasserlöslichen Edelmetallverbindung auf das Trägermaterial aufgetrocknet werden, um solche Verbindungen, die in bevozugter Ausführungsform in einer Konzentration, berechnet als Metall, von weniger als 4 Gew.-% in Wasser von 30°C gelöst werden können.

Diese Coatingverbindungen verfügen mithin über eine schlechte Wasserlöslichkeit, woraus die Suspension resultiert, die im ersten Schritt aufgebracht wird.

Zu den erfindungsgemäß einsetzbaren Coatingmaterialien gehören feinteilige anorganische Verbindungen metalloxidischer Art. Vorzugsweise eingesetzt werden SiO₂, Al₂O₃, TiO₂ und/oder ZrO₂. Besonders bevorzugt hiervon sind SiO₂ und Al₂O₃, ganz besonders bevorzugt ist SiO₂.

Zur Erzeugung der Schale des erfindungsgemäß herstellbaren Schalenkatalysators werden vorzugsweise Materialien mit einem mittleren Agglomeratgrößenbereich bis 15 µm, bevorzugt mit einer Agglomeratgröße im Bereich von 2 bis 10 µm eingesetzt. Ganz besonders bevorzugt ist der Einsatz von feinteiligen Kieselsäuren mit einem Agglomeratgrößenbereich von 3 bis 7 µm.

Größere Agglomerate/Partikel sind im allgemeinen für die Präparation nicht besonders vorteilhaft, da sie bedingt durch ihre Größe, keine homogene, fest haftende, abriebsbeständige Schale ausbilden können.

Die bevorzugt eingesetzten Metalloxide besitzen eine Oberfläche im Bereich von 50 bis 500 m²/g, bevorzugt 100 bis 300 m²/g sowie eine Stampfdichte (nach DIN/ISO 787/11XI, JISK 5101/18) im Bereich von 10 bis 800 g/l, bevorzugt 50 bis 500 g/l.

Ganz besonders zweckmäßig werden Kieselsäuren eingesetzt, welche die entsprechend genannten Oberflächen sowie Stampfdichten aufweisen.

Die unter der Verwendung der genannten Metalloxide präparierten Schalenkatalysatoren sind gemäß daran aufgenommener Rasterelektronenmikroskop-Aufnahmen überaus homogen, zeigen keine Schollenbildung mit Ausnahme von Makrobuchten (in der sich die aufgecoateten Materialien anhäufen können), an der Trägeroberfläche auch keine Rißbildung. Auf sehr hohen Vergrößerungen erkennt man eine weitgehende homogene körnige Struktur.

Die Schalendicke bei erfindungsgemäß hergestellten Schalenkatalysatoren kann über einen breiten Bereich recht gezielt gesteuert werden. Vorzugsweise liegt sie im Bereich von 0,1 bis 20 µm. Besonders bevorzugt sind Bereiche von 0,5 bis 10 µm. Äußerst zweckmäßig ist es, wenn die Schalendicke > 1 µm ist.

Das Verhältnis von in Wasser löslicher Edelmetallverbindung und von im wesentlichen nicht in Wasser löslicher Coatingverbindung in der auf das Trägermaterial aufzutrocknenden Suspension ist über einen weiten Bereich variabel. Besonders bevorzugt ist ein Verfahren der Erfindung, das sich dadurch auszeichnet, daß die in Wasser lösliche Edelmetallverbindung und die in Wasser im wesentlichen nicht lösliche Coatingverbindung, jeweils bezogen auf den Metallgehalt, in einem Gewichtsverhältnis von Edelmetallverbindung zu Coatingverbindung im Bereich von 0,1 : 1 bis 5 : 1 eingesetzt werden. Besonders zweckmäßig ist eine Variante, bei der die Edelmetallverbindung und die Coatingverbindung in einem Gewichtsverhältnis im Bereich von 0,5 : 1 bis 2 : 1 eingesetzt werden.

Weiterhin zweckmäßig ist es, wenn die Edelmetallverbindung, bezogen auf das Gesamtgewicht des Schalenkatalysators, in einem Gewichtsverhältnis, berechnet als Metall, im Bereich von 0,0001 : 1 bis 0,02 : 1 eingesetzt wird.

Außerdem ist es von besonderer Bedeutung, wenn die Coatingverbindung, bezogen auf das Gesamtgewicht des Schalenkatalysators und berechnet als Metall, in einem Gewichtsverhältnis im bevorzugten Bereich von 0,0005 : 1 bis 0,005 : 1 eingesetzt wird.

Der Gehalt an Metall, insbesondere Palladium oder Platin des Schalenkatalysators, wie er mit dem Verfahren gemäß der Erfindung herstellbar ist, liegt bevorzugt bei höchstens 1 Gew.-% Metall, bevorzugt im Bereich zwischen 0,1 und 0,5 Gew.-% Metall. Metallgehalte, insbesondere Palladiumgehalte, unter 0,1 Gew.-% sind nicht sinnvoll, da der Katalysator in Folge Abriebsbelastungen im Realeinsatz unter Prozeßbedingungen Edelmetall verlieren könnte und somit bei zu geringen Edelmetallgehalten eine zu kurze Einsatzdauer aufweisen würde. Höhere Edelmetallgehalte als 1 Gew.-% sind ebenfalls nicht besonders sinnvoll, da dies bei den vorliegenden Schalenkatalysatoren in Folge einer sehr geringen Oberfläche des Trägermaterials zu unnötigen Lagenanhäufungen des Edelmetalls unter Haftungsverminderung führen könnte und auf diese Weise zudem kein gesteigertes Leistungspotential verzeichnet wird. Der Gehalt an Coatingmaterialien, in den gemäß der Erfindung herstellbaren Schalenpräparationen liegt bevorzugt im Bereich von 0,05 bis 1 Gew.-%, ausgedrückt als Metall. Besonders bevorzugt liegt der Gehalt bei 0,05 bis 0,5 Gew.-%, ausgedrückt als Metall, bezogen auf das Gesamtkatalysatorgewicht. Bei diesen Größenordnungen des Zusatzes des bevorzugten feinteiligen Coatingmaterials ergibt sich ein überraschend guter Haftungseffekt.

Gemäß der zweiten Stufe des erfindungsgemäßen Verfahrens wurden die Schalenformulierungen in einem reduktiv wirksamen Gasstrom zu aktivierten Schalenkatalysatoren umgesetzt. Diese Behandlung besteht in der selektiven Reaktion einer der Legierungskomponenten mit einem Gas oder Gasgemisch bei höherer Temperatur.

Insbesondere ist es bevorzugt, daß ein Wasserstoff aufweisender Gasstrom zur Aktivierung eingesetzt wird.

Zur Verbesserung der Haftung zwischen Träger und aufzutrocknendem Material ist es möglich dem Fachmann an sich geläufige Haftungsvermittler, z. B. Wasserglas, zuzusetzen. Diese werden der Suspension zugegeben und im selben Verfahrensschritt auf den Träger aufgebracht.

Genauso gut ist es möglich, daß der Suspension zusätzliche Dotierungsverbindungen zugesetzt werden, um bestimmte Beeinflußungen der Aktivität/Selektivität des Katalysators zu ermöglichen.

Gegenstand der Erfindung ist auch die Verwendung des gemäß der hierin beschriebenen Vorgehensweise erhältlichen Schalenkatalysatoren zu selektiven Hydrierung von Acetylen in Gasströmen. Insbesondere vorteilig wird der erfindungsgemäß herstellare Schalenkatalysator in Gasströmen bei der Reinigung von Chlorwasserstoffgas im Vinylchloridprozeß eingesetzt.

Vorzugsweise wird also innerhalb der Erfindung ein Schalenkatalysator, hergestellt durch Auftrocknung einer Suspension aus einer Edelmetallsalzlösung, vorzugsweise Palladiumsalzlösung, mit einem feinteiligen Material, vorzugsweise Kieselsäure und anschließender Reduktion mit einem reduktiv wirksamen, bevorzugt wasserstoffhaltigen Gas, erhalten, der auf Trägermaterialien mit geringer Oberfläche basiert und der sich durch eine hohe Aktivität und Selektivität bei hohen Raumgeschwindigkeiten bis 8000/h, vorzugsweise im Bereich 2000 - 6000/h, bei der Reinigung eines Chlorwasserstoffgasstroms durch selektive Hydrierung des in ihm enthaltenen Acetylens zu Ethylen auszeichnet.

Die Prozeßbedingungen sind dabei besonders zweckmäßig:
Temperaturbereich 100 - 200°C , vorzugsweise 100 - 160°C;
Druckbereich für Chlorwasserstoffgasstrom (Prozeßdruck) 1 bis 15 bar abs., vorzugsweise 6 - 12 bar, abhängig vom Prozeßdruck für die Oxychlorierung;
Verweilzeit 2 - 15 sec. (bei Betriebsbedingungen und Leerrohr);
Wasserstoff : Acetylen-Verhältnis 1:1 bis 6:1, vorzugsweise 2:1 bis 4:1;
Nachfolgend wird die Erfindung an Hand von Beispielen unter Bezugnahme auf die beigefügten Abbildungen 1 - 3 weiter erläutert.

In den Abbildungen zeigen
- Abb. 1:: Umsatz-/Selektivitäts-/Abriebs-Verhalten des erfindungsgemäßen Schalenkatalysators in Abhängigkeit von dem Coatingmaterialanteil;
- Abb. 2:: Umsatz-/Selektivität des Katalysators gemäß Vergleichsbeispiel 1 in Abhängigkeit von der Raumgeschwindigkeit;
- Abb. 3:: Umsatz-/Selektivität des erfindungsgemäßen Katalysators gemäß Beispiel 2 (Schalenkatalysator) in Abhängigkeit von der Raumgeschwindigkeit;

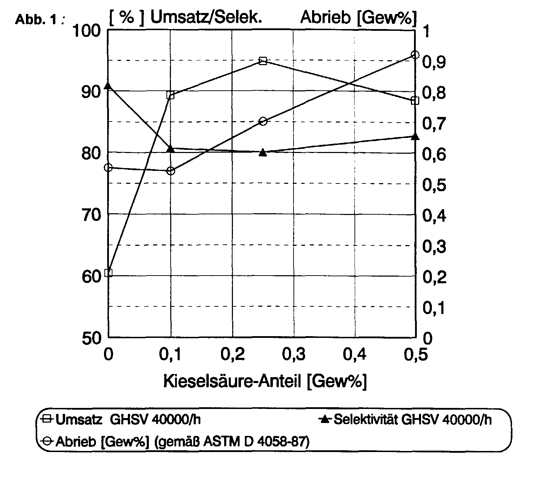

Abb. 1 zeigt die Abhängigkeit des Leistungspotentials (Umsatz/Selekltivität) und des Abriebs eines Schalenkatalysators gemäß der Erfindung, präpariert mit einer Palladiumsalzlösung auf einem SiO₂-Granulat der Körnung 3 - 5 mm, von dem Gehalt an Kieselsäure mit einer mittleren Agglomeratgröße von 7 um. Die Umsatz- und Selektivitätsdaten wurden in einer Labortestapparatur bei 130°C ermittelt. Das verwendete Testgas hatte folgende Zusammensetzung:
0,5 % Wasserstoff / 0,2 % Acetylen / 99,3 % Stickstoff.

Die Abbildung zeigt deutlich, daß bei Gehalten von > 0,25 Gew.-% Kieselsäure eine deutliche Abriebssteigerung auftritt, ohne das wesentliche Umsatz- und Selektivitätszugewinne zu verzeichnen sind. Ab Kieselsäuregehalten von 0,1 Gew.-% sind bereits wesentliche Umsatzsteigerungen bei erhöhter Raumgeschwindigkeit gegenüber einer Katalysatorpräparation ohne Kieselsäurezusatz zu verzeichnen. Somit liegt der besonders bevorzugte Kieselsäureanteil, bezogen auf das Gesamtkatalysatorgewicht des beispielhaften Katalysators, bei 0,1 - 0,3 Gew.-% der bevorzugt verwendeten Kieselsäure.

Ein Schalenkatalysator der beschriebenen besonders bevorzugten Zusammensetzung von 0,1 - 1 Gew.-% Pd und 0,1 - 1 Gew.-% Kieselsäure auf einem nicht porösen SiO₂-Träger der Körnung 3 - 5 mm und der geringen Oberfläche von < 1m²/g zeichnet sich durch ein sehr hohe Aktivität und Selektivität bei Raumgeschwindigkeiten bis 8000/h, bevorzugt 2000 - 6000/h, bei der beispielhaft angewendeten selektiven Acetylenhydrierung in einem Chlorwasserstoffgasstrom unter den beschriebenen Prozeßbedingungen aus.

Die gesteigerte Aktivität des erfindungsgemäßen Katalysators ist ein Resultat aus der verbesserten Feinverteilung des in die vorzugsweise aufgebrachte Kieselsäureschale integrierten Edelmetalls. Die Kieselsäure bewirkt dabei eine diesen Feinverteilungseffekt fördernde Oberflächenvergrößerung. Dagegen sind bei porösen imprägnierten Trägermaterialien die zusätzlichen oberflächlichen Trägerreaktivzentren ein zusätzliches Potential für unerwünschte Nebenreaktionen.

Der Schalenkatalystor der vorliegenden Erfindung besitzt ein erweitertes Aktivitätspotential. Für die beispielhaft gewählte Anwendung in der Chlorwasserstoffgasreinigung werden hohe Acetylenumsätze bis in einen GHSV-Bereich von 8000/h erreicht. Die Selektivitäten zu Ethylen liegen dabei über 60 %.

Bei der Präparation der Schalenkatalysatoren der vorliegenden Erfindung erfolgt die Auftrocknung der Suspension aus Edelmetallsalz, vorzugsweise einem Palladiumsalz, und feinteiligem Coatingmaterial, vorzugsweise Kieselsäure, unter letztendlicher Ausbildung einer homogenen Schale in bewegter Schüttung; d.h. die Suspension aus Edelmetallsalz und bevorzugt Kieselsäure wird zusammen mit dem Träger gut durchmischt und das Lösungswasser unter äußerer Beheizung destillativ entfernt.

Durch die Herstellungsmethodik und die Ausbildung einer oberflächlichen Schale sind zudem diverse Dotierungs- und haftungsvermittelnde Zusätze leicht möglich mit der entsprechenden Auswirkungen auf die Schalenmodifikation und das Leistungsverhalten. Bevorzugte Dotierungszusätze sind aus der Literatur bekannt (z.B. D.L.Trimm, Design of Industrial Catalysts, Elsevier Scientific Publ. Comp., 1980, S.229 ff).

### Herstellung des Katalysators im technischen Maßstab

### Vergleichsbeispiel 1:

### -Standardkatalysator ohne Kieselsäurezusatz-

Die Herstellung dieses Katalysators erfolgt in bewegter Schüttung. Geätztes und säurefrei gewaschenes SiO₂-Granulat wird in der Präparationstrommel vorgelegt. Unter kontinuierlich übergleitetem Stickstoffstrom wird die entsprechende Menge (0,15 Gew.-% Pd bezogen auf Katalysatorgewicht) an Palladiumchloridlösung in die Trommel zugegeben. Durch Zuleitung von Druckdampf (140°C) in den Außenmantel der Präparationstrommel wird die Palladiumsalzlösung bei rotierender Trommel auf den Träger aufgetrocknet. Die Reduktion erfolgt durch Überleiten eines überschüssigen Wasserstoffgasstroms in periodisch rotierender Präparationstrommel. Der Wasserstoffgastrom wird durch einen Stickstoffgasstrom in anschließenden Abkühlphase ersetzt.

Der nach dieser Präparationsmethodik erhaltene Katalysator besitzt eine grau-glänzende überaus dünne äußere Edelmetallschale von < 1 µm.

### Beispiel 2:

### - Herstellung des Schalenkatalysators -

In Abweichung zur Präparation des Standardkatalysators im Beispiel 1 wird der Schalenkatalysator durch Vorlage von 0,5 t trockenen SiO₂-Granulats begonnen. Danach wird die 0,25 Gew.-% des Katalysators entsprechende Menge an trockener Kieselsäure in die Präparationstrommel vorgelegt.

Es wird die 0,15 Gew.-% des Katalysators entsprechende Menge an Palladiumnitratlösung in die Präparationstrommel zugegeben. Nach einer Einwirkzeit unter Stickstoffüberleitung in rotierender Trommel schließt sich Auftrocknung bei 140°C gemäß Beispiel 1 an. Die Reduktion erfolgt durch Zuleitung von Formiergas (5 % Wasserstoff in Stickstoff) in die Schüttung des aufgetrockneten Katalysators.

Der erhaltene Katalysator ist tiefschwarz und matt.In Tabelle 1 sind die in Herstellungsbeispiel 1 und 2 beschriebenen Katalystoren hinsichtlich ihrer physikalischen Eigenschaften gegenübergestellt.

**Tabelle 1**

| **Parameter:** | **Katalysator gemäß Vergleichsbeispiel 1 -Standardkatalysator-** | **Katalysator gemäß Beispiel 2 -Schalenkatalysator-** |
|---|---|---|
| Pd-Gehalt [Gew.-%] | 0,15 | 0,15 |
| Kieselsäure-Anteil [Gew.-%] | 0 | 0,25 |
| Aussehen | grau/glänzend | matt/schwarz |
| Abtrieb [Gew.-%] ASTM D 4058-87 | 0,2 - 0,3 | 0,4 - 0,5 |
| BET-Oberfläche [m²/g] | keine Adsorption | < 1 |
| CO-Adsorption [10⁻² ml/g] | 0,3 | 1,25 |

### Leistungsvergleich:

### Anwendungsbeispiel I

### Vergleichsversuche 1 und 2

Die unter Vergleichsbeispiel 1 und Beispiel 2 beschriebenen Katalysatoren wurde in einer Pilottestanlage auf ihr Umsatz- und Selektivitätspotential hin getestet.

Die Pilottestanlage ist als Bypass-Reaktoranlage an eine bestehende Hydriereinheit zur Reinigung des Chlorvasserstoffgasstroms innerhalb der Vinylchloridproduktionsanlage angegliedert, so daß ein bis zu 2000 ppm Acetylen enthaltender realer Chlorwasserstoffgasstrom direkt auf die Katalysatoren angewendet werden kann. Es sind zwei Reaktoren parallel installiert, so daß in einem Reaktor ein Referenzkatalysator getestet werden kann, während im parallel geschalteten Reaktor Neuentwicklungen eingebracht werden können. Somit ist ein direkter Vergleich bei schwankenden Anlagenbedingungen und Qualitäten des Chlorwasserstoffs möglich. Die Reaktoren sind so ausgelegt, daß sie mit 2 l Katalysator befüllt werden können. Sie werden über Heizschlangen mit Druckdampf beheizt. Wasserstoff ist über Rotameter flexibel zudosierbar und jeder Reaktor verfügt über eine Temperaturmessung (mittig im Katalysatorbett), die zur Temperaturregelung herangezogen werden kann.

Der maximal zuleitbare Volumenstrom an Chlorwasserstoff beträgt pro Reaktor 16 Nm³/h. Das Reaktionsgas nach den Reaktoren wird separat einer gaschromatographischen Analytik zugeführt.

### Leistungsvergleich

Die in Vergleichsbeispiel 1 und Beispiel 2 beschriebenen Katalysatoren wurden mehrere Monate in der Pilottestanlage unter variierenden Parametern getestet. Anspruch des primären Leistungsvergleichs war u.a. die Fixierung des Umsatzes und die Erkundung der korrespondierenden Raumgeschwindigkeit als direktes Leistungsmaß. Die vergleichbare Einstellungen sind in Tabelle 2 und 3 zusammengestellt.

**Tabelle 2**

| Einstellung I: | | |
|---|---|---|
| **Vergleichsgröße:** | **Katalysator gemäß Vergleichsbeispiel 1 -Standardkatalysator- Reaktor A** | **Katalysator gemäß Beispiel 2 -Schalenkatalysator- Reaktor B** |
| Laufzeit [Wochen] | 8 | 8 |
| Temperatur | 130 | 130 |
| H₂ : C₂H₂-Verhältnis | 2 | 2 |
| Umsatz C₂H₂ [%] | 90 | 91 |
| Selektivität zu C₂H₄ [%] | 51 | 63 |
| **GHSV [1/h]** | **750** | **4500** |

**Tabelle 3**

| Einstellung II: | | |
|---|---|---|
| **Vergleichsgröße:** | **Katalysator gemäß Vergleichsbeispiel 1 -Standardkatalysator- Reaktor A** | **Katalysator gemäß Beispiel 2 -Schalenkatalysator- Reaktor B** |
| Laufzeit [Wochen] | 12 | 12 |
| Temperatur | 130 | 135 |
| H₂ : C₂H₂-Molverhältnis | 2 | 2 |
| Umsatz C₂H₂ [%] | 70 | 73 |
| Selektivität zu C₂H₄ [%] | 52 | 61 |
| **GHSV [1/h]** | **1500** | **6000** |

Der in Tabelle 2 und 3 dargestellte Leistungsvergleich bei fixiertem Umsatz zeigt deutlich das erhöhte Leistungspotential des Schalenkatalysators gemäß dieser Erfindung in dem Anwendungsfall der Chlorwasserstoffgasreinigung. Der Schalenkatalysator kann bei 4 - 6 mal höheren Raumgeschwindigkeiten bei gleichzeitigen Ethylenselektivitätsgewinn eingesetzt werden.

### Umsatz-/Selektivitätsverhalten

Das Umsatz und Selektivitätsverhalten der im Beispiel 1 und 2 beschriebenen Katalysatoren wurden bei verschiedenen Raumgeschwindigkeiten erfaßt und in Tabelle 4 zusammengestellt.

**Tabelle 4**

| Grundeinstellung: Temperatur (Schüttungsmitte): 130°C ; H₂ : C₂H₂ Molverhältnis = 2 | | | | | |
|---|---|---|---|---|---|
| **Katalysator gemäß Vergleichsbeispiel 1 - Standardkatalysator- Reaktor A** | | | **Katalysator gemäß Beispiel 2 - Schalenkatalysator- Reaktor B** | | |
| GHSV | Umsatz von C₂H₂ [%] | Selektivität zu C₂H₄ [%] | GHSV | Umsatz von C₂H₂ [%] | Selektivität zu C₂H₄ [%] |
| 750 | 90 | 52 | | | |
| 1000 | 82 | 57 | | | |
| 1500 | 70 | 52 | | | |
| 2250 | 60 | 64 | 2250 | 100 | 39 |
| | | | 4500 | 91 | 63 |
| 6000 | 16 | 60 | 6000 | 73 | 61 |

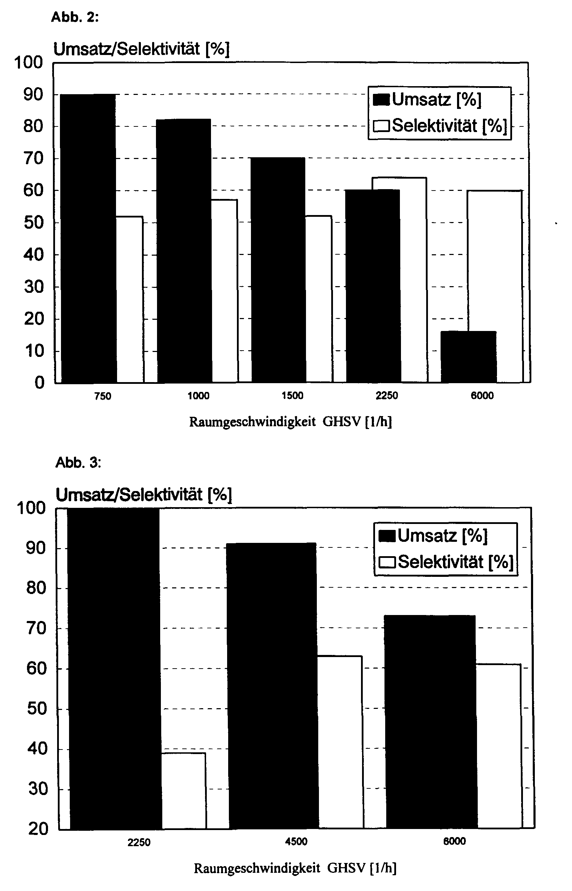

### Graphische Darstellung in Abb.2 und 3

Das Umsatz-/Selektivitätsverhalten des erfindungsgemäßen Schalenkatalysators zeigt, daß gegenüber dem Standardkatalysator (gemäß Stand der Technik), weitaus höhere Raumgeschwindigkeiten angewendet werden können, ohne das Umsatzeinbrüche auftreten. Im hohen GHSV-Bereich ab 4000/h ergeben sich sehr vorteilhafte, bevorzugte Umsatz- und Selektivitätskonstellationen.

## Patentansprüche

1. Verfahren zur Herstellung eines Schalenkatalysators, bei dem ein nicht poröses anorganisches Trägermaterial, welches eine BET-Oberfläche von weniger als 80 m²/g aufweist, mit einer katalytisch wirksamen Schale versehen wird, dadurch gekennzeichnet, daß zunächst in einem Schritt eine Suspension aus wenigstens einer in Wasser löslichen Edelmetallverbindung und einer im wesentlichen nicht wasserlöslichen Coatingverbindung auf das Trägermaterial aufgetrocknet wird und anschließend das resultierende schalenförmig beschichtete Trägermaterial in einem reduktiv wirksamen Gasstrom aktiviert wird.

2. Verfahren nach Anspruch 1,
**dadurch gekennzeichnet,**
daß als Trägermaterialien Formkörper aus Glas, Quarz, Keramik, Siliciumdioxid, Aluminiumoxid, Graphit, Formkohlen, Metall oder Steatit eingesetzt werden.

3. Verfahren nach Anspruch 2,
**dadurch gekennzeichnet,**
daß Formkörper auf Basis SiO₂ und/oder Al₂O₃ eingesetzt werden.

4. Verfahren nach einem oder mehreren der vorhergehenden Ansprüche,
**dadurch gekennzeichnet,**
daß Trägermaterialien in Form von Hohlextrudaten, Vollextrudaten, Kugeln, Granalien, Tabletten und/oder Strängen eingesetzt werden.

5. Verfahren nach einem oder mehreren der vorhergehenden Ansprüche,
**dadurch gekennzeichnet,**
daß Trägermaterialien mit Teilchendurchmesser im Bereich von 0,5 bis 50 mm eingesetzt werden.

6. Verfahren nach einem oder mehreren der vorhergehenden Ansprüche,
**dadurch gekennzeichnet,**
daß Trägermaterialien mit einer BET-Oberfläche von < 10 m²/g eingesetzt werden.

7. Verfahren nach einem oder mehreren der vorhergehenden Ansprüche,
**dadurch gekennzeichnet,**
daß Trägermaterialien mit einem Porenvolumen < 0,5 ml/g eingesetzt werden.

8. Verfahren nach Anspruch 7,
**dadurch gekennzeichnet,**
daß Trägermaterialien mit einem Porenvolumen von < 0,1 ml/g eingesetzt werden.

9. Verfahren nach einem oder mehreren der vorhergehenden Ansprüche,
**dadurch gekennzeichnet,**
daß Trägermaterialien mit einem Fe₂O₃-Gehalt < 0,5 Gew.-% eingesetzt werden.

10. Verfahren nach einem oder mehreren der vorhergehenden Ansprüche,
**dadurch gekennzeichnet,**
daß wasserlösliche Verbindungen von Ru, Rh, Pd, Ag, Os, Ir, Pt und/oder Au eingesetzt werden.

11. Verfahren nach Anspruch 10,
**dadurch gekennzeichnet,**
daß Oxide, Hydroxide, Carbonate, Halogenide, Nitrate, Salze organischer Säuren und/oder Komplexverbindungen der Edelmetalle eingesetzt werden.

12. Verfahren nach einem oder mehreren der vorhergehenden Ansprüche,
**dadurch gekennzeichnet,**
daß die wasserlösliche Edelmetallverbindung als wäßrige Lösung enthaltend die Edelmetallverbindung, berechnet als Metall, in einer Konzentration von > 1 Gew.-%, eingesetzt wird.

13. Verfahren nach Anspruch 12,
**dadurch gekennzeichnet,**
daß die wasserlösliche Edelmetallverbindung als wäßrige Lösung eingesetzt wird, welche die Edelmetallverbindung in einer Konzentration von > 5 Gew.-% enthält.

14. Verfahren nach einem oder mehreren der vorhergehenden Ansprüche,
**dadurch gekennzeichnet,**
daß Edelmetallverbindungen eingesetzt werden, die in einer Konzentration, berechnet als Metall von wenigstens 0,01 Gew.-% in Wasser von 30°C gelöst werden können.

15. Verfahren nach einem oder mehreren der vorhergehenden Ansprüche,
**dadurch gekennzeichnet,**
daß als Coatingmaterialien oxidische Verbindungen von Metallen eingesetzt werden, die in einer Konzentration, berechnet als Metall, von weniger als 4 Gew.-% in Wasser von 30°C gelöst werden können.

16. Verfahren nach Anspruch 15,
**dadurch gekennzeichnet,**
daß SiO₂, Al₂O₃, TiO₂ und/oder ZrO₂ eingesetzt werden.

17. Verfahren nach Anspruch 16,
**dadurch gekennzeichnet,**
daß Metalloxide mit einem mittleren Agglomeratgrößenbereich bis 15 µm eingesetzt werden.

18. Verfahren nach Anspruch 17,
**dadurch gekennzeichnet,**
daß Metalloxide mit einer Agglomeratgröße im Bereiche von 3 - 7 µm eingesetzt werden,

19. Verfahren nach einem oder mehreren der vorgenannten Ansprüche 15 bis 18,
**dadurch gekennzeichnet,**
daß Metalloxide mit einer BET-Oberfläche im Bereich von 50 - 500 m²/g eingesetzt werden.

20. Verfahren nach einem oder mehreren der vorgenannten Ansprüche 15 bis 19,
**dadurch gekennzeichnet,**
daß Metalloxide mit einer Stampfdichte im Bereich von 10 - 800 g/l eingesetzt werden.

21. Verfahren nach einem oder mehreren der vorgenannten Ansprüche,
**dadurch gekennzeichnet,**
daß die in Wasser lösliche Edelmetallverbindung und die in Wasser im wesentlichen nicht lösliche Coatingverbindung, jeweils bezogen auf den Metallgehalt, in einem Gewichtsverhältnis von Edelmetallverbindung : Coatingverbindung im Bereich von 0,1 : 1 bis 5 : 1 eingesetzt werden.

22. Verfahren nach Einspruch 21,
**dadurch gekennzeichnet,**
daß Edelmetallverbindung : Coatingverbindung in einem Gew.-Verhältnis im Bereich 0,5 : 1 bis 2 : 1 eingesetzt werden.

23. Verfahren nach einem oder mehreren der vorgenannten Ansprüche,
**dadurch gekennzeichnet,**
daß die Edelmetallverbindung, bezogen auf das Gesamtgewicht des Schalenkatalysators, in einem Gewichtsverhältnis, berechnet als Metall, im Bereich von 0,0001 : 1 bis 0,02 : 1, eingesetzt wird.

24. Verfahren nach einem oder mehreren der vorgenannten Ansprüche,
**dadurch gekennzeichnet,**
daß die Coatingverbindung, bezogen auf das Gesamtgewicht des Schalenkatalysators und berechnet als Metall, in einem Gewichtsverhältnis im Bereich von 0,0005 : 1 bis 0,04 : 1, eingesetzt wird.

25. Verfahren nach einem oder mehreren der vorgenannten Ansprüche,
**dadurch gekennzeichnet,**
daß ein Wasserstoff aufweisender Gasstrom zur Aktivierung eingesetzt wird.

26. Verfahren nach einem oder mehreren der vorgenannten Ansprüche,
**dadurch gekennzeichnet,**
daß der auf den Träger aufzutrocknenden Suspension Haftungsvermittler zugesetzt werden.

27. Verfahren nach einem oder mehreren der vorgenannten Ansprüche,
**dadurch gekennzeichnet,**
daß der Suspension zusätzliche Dotierungsverbindungen zugesetzt werden.

28. Verwendung des nach den vorhergehenden Ansprüchen erhaltenen Schalenkatalysators zur selektiven Hydrierung von Acetylen in Gasströmen, insbesondere von Chlorwasserstoffgas im Vinylchloridprozess.
